# EUROPEAN PATENT SPECIFICATION

(11) **EP 3 615 010 B1**
(45) Date of publication and mention of the grant of the patent: **21.08.2024**
(21) Application number: 18791035.1
(22) Date of filing: 26.04.2018
(51) Int. Cl.: A61K 31/44, A61K 33/24, A61K 38/00, A61P 25/08

(54) **METHODS FOR TREATING DRAVET SYNDROME**
VERFAHREN ZUR BEHANDLUNG DES DRAVET-SYNDROMS
MÉTHODES DE TRAITEMENT DU SYNDROME DE DRAVET

(30) Priority: 26.04.2017 US 201762490357 P
(43) Date of publication of application: 04.03.2020
(73) Proprietor: Cavion, Inc., Charlottesville, VA 22902 (US)
(72) Inventor: MARICICH, Yuri, Charlottesville VA 22901 (US); NEWBOLD, Evan, Charlottesville VA 22902 (US)
(74) Representative: Carpmaels & Ransford LLP
(86) International application number: PCT/US2018/029610
(87) International publication number: WO 2018/200844

(56) References cited:
- WO-A1-2016/203239
- WO-A1-2017/070680
- WO-A2-2012/094615
- IANNETTI P ET AL: "Addition of verapamil in the treatment of severe myoclonic epilepsy in infancy", EPILEPSY RESEARCH, vol. 85, no. 1, 1 July 2009 (2009-07-01), pages 89 - 95, XP026171452, ISSN: 0920-1211, [retrieved on 20090320], DOI: 10.1016/J.EPLEPSYRES.2009.02.014
- SO-HEE EUN ET AL: "Comparative trial of low- and high-dose zonisamide as monotherapy for childhood epilepsy", SEIZURE, BAILLIERE TINDALL, LONDON, GB, vol. 20, no. 7, 1 April 2011 (2011-04-01), pages 558 - 563, XP028247499, ISSN: 1059-1311, [retrieved on 20110407], DOI: 10.1016/J.SEIZURE.2011.04.005
- IINUMA K ET AL: "Clinical efficacy of zonisamide in childhood epilepsy after long-term treatment: a postmarketing, multi-institutional survey", SEIZURE, BAILLIERE TINDALL, LONDON, GB, vol. 13, 1 December 2004 (2004-12-01), pages S34 - S39, XP004613926, ISSN: 1059-1311, DOI: 10.1016/J.SEIZURE.2004.04.003
- KEENE D L ET AL: "FLUNARIZINE AS A SUPPLEMENTARY MEDICATION IN REFRACTORY CHILDHOOD EPILEPSY A DOUBLE-BLIND CROSSOVER STUDY", CANADIAN JOURNAL OF NEUROLOGICAL SCIENCES, vol. 16, no. 2, 1989, pages 191 - 193, XP002801434, ISSN: 0317-1671
- DATABASE BIOSIS [online] BIOSCIENCES INFORMATION SERVICE, PHILADELPHIA, PA, US; 1989, KEENE D L ET AL: "FLUNARIZINE AS A SUPPLEMENTARY MEDICATION IN REFRACTORY CHILDHOOD EPILEPSY A DOUBLE-BLIND CROSSOVER STUDY", Database accession no. PREV198988066002
- KEENE D L ET AL: "FLUNARIZINE AS A SUPPLEMENTARY MEDICATION IN REFRACTORY CHILDHOOD EPILEPSY A DOUBLE-BLIND CROSSOVER STUDY", CANADIAN JOURNAL OF NEUROLOGICAL SCIENCES, vol. 16, no. 2, 1989, pages 191 - 193, XP002801434, ISSN: 0317-1671
- POWELL ET AL.: "Low threshold T-type calcium channels as targets for novel epilepsy treatments", BRITISH JOURNAL OF CLINICAL PHARMACOLOGY, vol. 77, 22 April 2014 (2014-04-22), pages 729 - 739, XP055528476
- CASILLAS-ESPINOSA ET AL.: "Z944, a Novel Selective T-Type Calcium Channel Antagonist Delays the Progression of Seizures in the Amygdala Kindling Model", PLOS ONE, vol. 10, no. 8, 14 August 2015 (2015-08-14), pages 1 - 12, XP055372894

## Description

### CROSS-REFERENCE TO RELATED APPLICATIONS

This application claims the benefit of U.S. Patent Application Serial No. 62/490,357, filed on April 26, 2017.

### TECHNICAL FIELD

This disclosure relates to treatment of disease by administering pharmaceutical compounds. In particular, the disclosure relates to the treatment of Dravet Syndrome by administering a T-type calcium channel antagonist.

### BACKGROUND

T-type calcium channels are low-voltage activated calcium channels that open during membrane depolarization and mediate calcium influx into cells after an action potential or depolarizing signal. T-type calcium channels known to be present within cardiac and smooth muscle, and also are present in many neuronal cells within the central nervous system. T-type calcium channels (transient opening calcium channels) are distinct from L-type calcium channels (Long-Lasting calcium channels) due to their ability to be activated by more negative membrane potentials, their small single channel conductance, and their non-responsiveness to traditional calcium channel antagonist drugs, targeting L-type calcium channels.

T-type calcium channels open following small membrane depolarizations. T-type calcium channels have been primarily studied in the context of neuronal and cardiomyocyte function, and have been implicated in hyperexcitability disorders, such as epilepsy and cardiac dysfunction. Voltage gated calcium channels are not generally expressed in non-excitable cells, but there is evidence that T-type calcium channels are expressed in cancer cells of non-excitable lineages.

T-type calcium channels are activated and inactivated by small membrane depolarizations, and display slow deactivation rates. Thus, these channels can carry depolarizing current at low membrane potentials and mediate cellular "window" currents, which occur within the voltage overlap between activation and steady state inactivation at low or resting membrane potentials. T-type calcium channels can maintain window current at non-stimulated or resting membrane potentials, thereby allowing a sustained inward calcium current carried by a portion of channels that are not inactivated. Mediation of window current allows T-type calcium channels to regulate intracellular calcium levels, both in electrically firing cells such as neurons, and in non-excitable tissues, under non-stimulated or resting cellular conditions.

Voltage-gated calcium channels are made up of several subunits. The α₁ subunit is the primary subunit that forms the transmembrane pore of the channel. The α₁ subunit also determines the type of calcium channel. The β, α₂δ, and γ subunits, present in only some types of calcium channels, are auxiliary subunits that play secondary roles in the channel. The ai subunit is composed of four domains (I-IV), with each domain containing 6 transmembrane segments (S1-S6), and hydrophobic loops between the S5 and S6 segments of each domain form the pore of the channel. Sub-types of the T-type calcium channel are defined by the specific α₁ subunit as shown in Table 1.

**Table 1. T-type Calcium Channel Sub-Types**

| Designation | α₁ subunit | Gene |
|---|---|---|
| Cav3.1 | α₁G | CACNA1G |
| Cav3.2 | α₁H | CACNA1H |
| Cav3.3 | α₁I | CACNA1I |

Dravet syndrome is a severe from of epilepsy, also known as epilepsy with polymorphic seizures, polymorphic epilepsy in infancy (PMEI) or severe myoclonic epilepsy of infancy (SMEI). It is a rare genetic disorder that affects an estimated 1 in every 20,000-40,000 births.

The syndrome is characterized by prolonged febrile and non-febrile seizures beginning within the first year of a child's life. Children with Dravet syndrome typically experience a lagged development of language and motor skills, hyperactivity and sleep difficulties, chronic infection, growth and balance issues, and difficulty relating to others. The effects of this disorder do not diminish over time. As the disease progresses other seizure types such as myoclonic and partial seizures, psychomotor delay, and ataxia occur. The disease is also characterized by cognitive impairment, behavioral disorders such as hyperactivity and impulsiveness, and motor deficits. Dravet syndrome is also associated with sleep disorders including somnolence and insomnia. Dravet syndrome is also associated with premature death.

In most cases the genetic mutations in Dravet syndrome are not hereditary with the mutated gene being found for the first time in the affected patient. The disease involves defects in the sodium channel genes known as SCN1A and SCN2A. A mutation in either of these two genes will cause an individual to develop dysfunctional sodium channels, which are crucial in the pathway for sending chemical signals in the brain, causing the phenotypic display of myoclonic epilepsy from the individual. A properly functioning channel would respond to a voltage difference across the membrane and form a pore through which only sodium ions can pass. The influx of sodium induces the generation of action potential by temporarily changing the charge of the cell. When the gene is mutated, the eventually translated protein improperly folds its pore segment within the cell membrane because it has different amino acid chemistry, which renders the channel inactive. It is also possible for a mutation to reduce the number of channels produced by an individual, which leads to the development of Dravet syndrome.

The SCN1A gene is the most clinically relevant. Typically, a missense mutation in either the S5 or S6 segment of the sodium channel pore results in a loss of channel function and the development of Dravet syndrome. A heterozygous inheritance of an SCN1A mutation is all that is necessary to develop a defective sodium channel; patients with Dravet syndrome will still have one normal copy of the gene.

Seizures in Dravet syndrome are difficult to manage, but they can be somewhat reduced by anticonvulsant drugs. Because the course of the disorder and the severity of seizures varies from individual to individual, a standard treatment protocol is difficult to establish. Certain anticonvulsant drugs such as those classed as sodium channel blockers, such as carbamazepine, gabapentin, lamotrigine, and phenytoin, can make seizures worse in most Dravet patients. Current treatments for Dravet syndrome are therefore usually insufficient.

There is therefore a need for new and effective treatments of Dravet Syndrome.

### SUMMARY

The present invention is defined in the appended claims.

The present disclosure provides a method of treating Dravet syndrome (i.e., severe myoclonic epilepsy of infancy (SMEI)). The method includes administering to a subject in need of such treatment a therapeutically effective amount of a T-type calcium channel antagonist. Also provided is the use of a T-type calcium channel antagonist for treating Dravet syndrome. The disclosure also provides the use of T-type calcium channel antagonist in the manufacture of a medicament for treating Dravet syndrome.

In some aspects of the disclosure the T-type calcium channel antagonist is a calcium channel antagonist that selectively targets T-type calcium channels.

In some aspects of the disclosure the T-type calcium channel antagonist is a calcium channel antagonist that selectively targets T-type calcium channels over L-type calcium channels.

In some aspects of the disclosure the T-type calcium channel antagonist is a small molecule.

In some aspects of the disclosure the T-type calcium channel antagonist is an antibody.

In some aspects of the disclosure the T-type calcium channel antagonist is a siRNA.

In some aspects of the disclosure , the T-type calcium channel antagonist selectively targets CaV3.1.

In some aspects of the disclosure the T-type calcium channel antagonist selectively targets CaV3.2.

In some aspects of the disclosure, the T-type calcium channel antagonist selectively targets CaV3.3.

In some aspects of the disclosure the T-type calcium channel antagonists antagonize aT-type calcium channel in a cell when the membrane potential of the cell is in the range from about -60 mV to about -30 mV, e.g., about -40 mV.

In some aspects of the disclosure T-type calcium channel antagonist is selected from the group consisting of mibefradil, diltiazem, nifedipine, nitrendipine,nimodipine, niludipine, niguldipine, nicardipine, nisoldipine, amlodipine, felodipine, isradipine, ryosidine, gallopamil, verapamil, tiapamil, pimozide, thioridazine, NNC 55-0396, TTL-1177, anandamide, pimozide, penfluridol, clopimozide, fluspirilene, haloperidol, droperidol, benperidol, triperidol, melperone, lenperone, azaperone, domperidone, antrafenine, aripiprazole, ciprofloxacin, dapiprazole, dropropizine, etoperidone, itraconazole, ketoconazole, levodropropizine, mepiprazole, naftopidil, nefazodone, niaprazine, oxypertine, posaconazole, trazodone, urpidil, vesnarinone, manidipine, nilvadipine, benidipine, efonidipine, flunarizine, anandamide, lomerizine, zonisamide, U-92032, tetralol, mibefradil, NNC 55-0396, TTA-A2, TTA-A8, TTA-P1, 4-aminomethyl-4-fluoropiperidine (TTA-P2), TTA-Q3, TTA-Q6, MK-5395 (CX-5395), MK-6526, MK-8998 (CX-8998), Z941, Z944, ethosuximide, phensuximide, mesuximide, desmethylmethsuximide, efonidipine, trimethadione, dimethadione, ABT-639, TTL-1177, KYSO5044, nickel, and kurtoxin, and combinations thereof.

According to the invention, the T-type calcium channel antagonist is MK-8998.

In some embodiments, the treatment comprises reducing or ameliorating at least one neurological symptom in the subject.

In some embodiments, the neurological symptom comprises one or more of seizure, hyperactivity, impulsiveness, autistic behavior, somnolence, insomnia, psychomotor delay, ataxia, cognitive impairment, neurological development, developmental delay, and impaired behavior.

In some embodiments, the treatment comprises reducing the frequency of seizure in the subject. In some embodiments, the treatment comprises reducing the severity of seizure in the subject. In some embodiments, the seizure is a febrile seizure. In some embodiments, the febrile seizure is a simple febrile seizure. In some embodiments, the febrile seizure is complex seizure. In some embodiments, the seizure is a myoclonic seizure. In some embodiments, the seizure is a partial seizure.

In some embodiments, the treatment comprises reducing the frequency of hyperactivity in the subject. In some embodiments, the treatment comprises reducing the severity of hyperactivity in the subject.

In some embodiments, the treatment comprises reducing the frequency of impulsiveness in the subject. In some embodiments, the treatment comprises reducing the severity of impulsiveness in the subject.

In some embodiments, the trearment comprises reducing the frequency of autistic behavior in the subject. In some embodiments, the treatment comprises reducing the severity of autistic behavior in the subject.

In some embodiments, the treatment comprises reducing the frequency of somnolence in the subject. In some embodiments, the treatment comprises reducing the severity of somnolence in the subject.

In some embodiments, the treatment comprises reducing the frequency of insomnia in the subject. In some embodiments, the treatment comprises reducing the severity of insomnia in the subject.

In some subjects, the treatment comprises reducing the psychomotor delay of the subject.

In some embodiments, the treatment comprises reducing the frequency of ataxia in the subject. In some embodiments, the treatment comprises reducing the severity of ataxia in the subject

In some embodiments, the treatment comprises reducing the severity of cognitive impairment in the subject. In some embodiments, the treatment comprises improving the cognition of the subject. In some embodiments, the treatment comprises improving the memory of the subject. In some embodiments, the treatment comprises improving the short-term memory of the subject In some embodiments, the treatment comprises improving the working memory of the subject. In some embodiments, the treatment comprises improving the long-term memory of the subject.

In some embodiments, the treatment comprises improving the neurological development of the subject.

In some subjects, the treatment comprises reducing the developmental delay of the subject.

In some embodiments, the treatment comprises reducing the frequency of impaired behavior in the subject. In some embodiments, the treatment comprises reducing the severity of impaired behavior in the subject.

In some embodiments, the treatment comprises prolonging survival in the subject. The treatment may reduce the risk of premature death or delay death.

In some embodiments, the selective T-type calcium channel antagonist substantially crosses the blood brain barrier. In other embodiments, the T-type calcium channel antagonist does not substantially cross the blood brain barrier.

In some embodiments, the treatment includes administering to the subject an additional therapeutic agent, which can be, *e.g.*, an additional T-type calcium channel inhibitor or an anticonvulsive agent.

In some embodiments, the anticonvulsive agent is selected from acetazolamide, clobazam, clonazepam, eslicarbazepine acetate, ethosuximide, lacosamide, levetiracetam, nitrazepam, oxcarbazepine, perampanel, piracetam, phenobarbital, pregabalin, primidone, retigabine, rufinamide, valproate, stiripentol, tiagabine, topiramate, vigabatrin, and zonisamide.

In some embodiments, the treatment includes administering an additional therapy, which can be selected, e.g., from the group consisting of a ketogenic diet, physical therapy, occupational therapy, communication therapy, and behavioral therapy.

Although methods and materials similar or equivalent to those described herein can be used in the practice or testing of the present invention, suitable methods and materials are described below.

Other features and advantages of the invention will be apparent from the following detailed description, and from the claims.

### DESCRIPTION OF THE DRAWINGS

FIG. 1 is a scheme showing the breeding of a Dravet model mouse with a Cav3.1 genetic knockout (KO) mouse to produce a heterozygous hybrid knock-out of CACNA1G in Dravet mouse model.
FIG. 2A is a plot showing the effect of the deletion of CACNA1G providing a protective benefit for spontaneous generalized tonic-clonic seizures in Dravet model mice.
FIG. 2B is a plot showing the effect of the deletion of CACNA1G providing a survival benefit in Dravet model mice.
FIG. 3 is a scheme showing a protocol for a hyperthermia induced seizure model in *Sen1a*^{+/-} mice to evaluate the effect of a selective Cav3 antagonist drug.

### DETAILED DESCRIPTION

The present invention is defined in the appended claims. The present disclosure describes that T-type voltage-gated calcium channels are involved in Dravet syndrome (*i*.*e*., severe monoclinic epilepsy in infants; SMEI). The present disclosure further describes that modulation of such T-type voltage-gated calcium channels can be effective for the treatment of Dravet syndrome.

### I. Definitions

Unless defined otherwise, all technical and scientific terms used herein have the same meaning as is commonly understood by one of ordinary skill in the art to which this disclosure belongs.

For the terms "e.g." and "such as," and grammatical equivalents thereof, the phrase "and without limitation" is understood to follow unless explicitly stated otherwise.

The singular forms "a," "an," and "the" include plural referents unless the context clearly dictates otherwise.

The term "about" means "approximately" (e.g., plus or minus approximately 10% of the indicated value).

The term "small molecule" means an organic compound with a molecular weight of about 1000 or less.

The term "subject," referring to the subject of treatment, means any animal, including mammals, e.g., human.

The phrase "therapeutically effective amount" refers to the amount of active compound or pharmaceutical agent that elicits the biological or medicinal response that is being sought in a tissue, system, animal, individual or human by a researcher, veterinarian, medical doctor or other clinician.

The term "treating" or "treatment" refers to one or more of (1) preventing a disease; e.g., preventing a disease, condition or disorder in an individual who may be predisposed to the disease, condition or disorder but does not yet experience or display the pathology or symptomatology of the disease; (2) inhibiting a disease; e.g., inhibiting a disease, condition or disorder in an individual who is experiencing or displaying the pathology or symptomatology of the disease, condition or disorder (*i.e.*, arresting further development of the pathology and/or symptomatology); and (3) ameliorating a disease; for example, ameliorating a disease, condition or disorder in an individual who is experiencing or displaying the pathology or symptomatology of the disease, condition or disorder (*i.e.*, reversing the pathology and/or symptomatology) such as decreasing the severity of disease or reducing or alleviating one or more symptoms of the disease.

The term "T-type calcium channel antagonists" refers to a substance that reduces the activity of T-type calcium channels, e.g., through binding to, or otherwise inhibiting or blocking activity of the channel, or through reducing the expression of T-type calcium channels.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

It is appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

The following abbreviations and symbols may be used in the present disclosure: DNA (deoxyribonucleic acid); dsRNA (double stranded RNA); g (gram); IC₅₀ (half maximal inhibitory concentration); kg (kilogram); mg (milligram); mRNA (messenger RNA); RNA (ribonucleic acid); RNAi (RNA interference); siRNA (small interfering RNA), wt (weight).

### II. Methods of Treatment

Provided herein are methods of treating Dravet syndrome in a subject in need thereof. The subject can include mice, rats, other rodents, rabbits, dogs, cats, swine, cattle, sheep, horses, primates, and humans. In some embodiments, the subject is a human. In some embodiments, the treating comprises reducing or ameliorating a neurological symptom associated with Dravet syndrome. In some embodiments, the neurological symptom comprises one or more of seizure, hyperactivity, impulsiveness, autistic behavior, somnolence, insomnia, psychomotor delay, ataxia, cognitive impairment, neurological development, developmental delay, and impaired behavior. In some embodiments, the method comprises administering a therapeutically effective amount of a T-type calcium channel antagonist as described herein, to the subject in need of the treatment.

In some embodiments, the treatment comprises reducing the frequency of seizure in the subject. In some embodiments, the treatment comprises reducing the severity of seizure in the subject In some embodiments, the seizure associated with Dravet syndrome is a febrile seizure. In some embodiments, the febrile seizure is a simple febrile seizure. In some embodiments, the febrile seizure is complex seizure. In some embodiments, the seizure associated with Dravet syndrome is a myoclonic seizure. In some embodiments, the seizure associated with Dravet syndrome is a partial seizure.

In some embodiments, the treatment comprises reducing the frequency of hyperactivity in the subject. In some embodiments, the treatment comprises reducing the severity of hyperactivity in the subject.

In some embodiments, the treatment comprises reducing the frequency of impulsiveness in the subject In some embodiments, the treatment comprises reducing the severity of impulsiveness in the subject.

In some embodiments, the treatment comprises reducing the frequency of autistic behavior in the subject. In some embodiments, the treatment comprises reducing the severity of autistic behavior in the subject.

In some embodiments, the treatment comprises reducing the frequency of somnolence in the subject. In some embodiments, the treatment comprises reducing the severity of somnolence in the subject.

In some embodiments, the treatment comprises reducing the frequency of insomnia in the subject. In some embodiments, the treatment comprises reducing the severity of insomnia in the subject.

In some subjects, the treatment comprises reducing the psychomotor delay of the subject.

In some embodiments, the treatment comprises reducing the frequency of ataxia in the subject. In some embodiments, the treatment comprises reducing the severity of ataxia in the subject.

In some embodiments, the treatment comprises reducing the severity of cognitive impairment in the subject. In some embodiments, the treatment comprises improving the cognition of the subject. In some embodiments, the treatment comprises improving the memory of the subject. In some embodiments, the treatment comprises improving the short-term memory of the subject. In some embodiments, the treatment comprises improving the working memory of the subject. In some embodiments, the treatment comprises improving the long-term memory of the subject.

In some embodiments, the treatment comprises improving the neurological development of the subject.

In some subjects, the treatment comprises reducing the developmental delay of the subject.

In some embodiments, the treatment comprises reducing the frequency of impaired behavior in the subject. In some embodiments, the treatment comprises reducing the severity of impaired behavior in the subject.

In some embodiments, the treatment comprises prolonging survival in the subject. The treatment may reduce the risk of premature death or delay death.

In some embodiments, the selective T-type calcium channel antagonist substantially crosses the blood brain barrier. In other embodiments, the T-type calcium channel antagonist does not substantially cross the blood brain barrier.

In some embodiments, the treatment includes administering to the subject an additional therapeutic agent, which can be, e.g., an additional T-type calcium channel inhibitor or an anticonvulsive agent.

The treatment can be administered at an effective dose for the particular compound. Examples of suitable doses include, in humans, include dosages in the range from about 1 mg to about 2000 mg, e.g., about 1 mg to about 2000 mg, about 2 mg to about 2000 mg, about 5 mg to about 2000 mg, about 10 mg to about 2000 mg, about 20 mg to about 2000 mg, about 50 mg to about 2000 mg, about 100 mg to about 2000 mg, about 150 mg to about 2000 mg, about 200 mg to about 2000 mg, about 250 mg to about 2000 mg, about 300 mg to about 2000 mg, about 400 mg to about 2000 mg, about 500 mg to about 2000 mg, about 1000 mg to about 2000 mg, about 1 mg to about 1000 mg, about 2 mg to about 1000 mg, about 5 mg to about 1000 mg, about 10 mg to about 1000 mg, about 20 mg to about 1000 mg, about 50 mg to about 1000 mg, about 100 mg to about 1000 mg, about 150 mg to about 1000 mg, about 200 mg to about 1000 mg, about 250 mg to about 1000 mg, about 300 mg to about 1000 mg, about 400 mg to about 1000 mg, about 500 mg to about 1000 mg, about 1 mg to about 500 mg, about 2 mg to about 500 mg, about 5 mg to about 500 mg, about 10 mg to about 500 mg, about 20 mg to about 500 mg, about 50 mg to about 500 mg, about 100 mg to about 500 mg, about 150 mg to about 500 mg, about 200 mg to about 500 mg, about 1 mg to about 250 mg, about 2 mg to about 250 mg, about 5 mg to about 250 mg, about 10 mg to about 250 mg, about 20 mg to about 250 mg, about 50 mg to about 250 mg, about 100 mg to about 250 mg, about 1 mg to about 100 mg, about 2 mg to about 100 mg, about 5 mg to about 100 mg, about 10 mg to about 100 mg, about 20 mg to about 100 mg, about 50 mg to about 100 mg. Doses can be, *e*.*g*., about 1 mg, about 2 mg, about 5 mg, about 10 mg, about 20 mg, about 50 mg, about 100 mg, about 150 mg, about 200 mg, about 250 mg, about 300 mg, about 400 mg, about 500 mg, about 1000 mg, about 1500 mg, or about 2000 mg. Doses can be less than about 2000 mg, less than about 1500 mg, less than about 1000 mg, less than about 5000 mg, less than about 400 mg, less than about 250 mg, less than about 200 mg, less than about 150 mg, less than about 100 mg, less than about 50 mg, less than about 20 mg or less than about 10 mg. Each of the doses can be doses that are administered at a frequency of once daily, twice daily, three times daily or four times daily, or less than once daily. Each of the doses can also be the dose that is administered to an adult with equivalent (scaled) dose being administered for pediatric patients.

The dose can be a dose that provides a plasma level (*e.g.*, a steady state or a maximum level) of about 100 ng/mL, about 200 ng/mL, 500 ng/mL, about 1 µg/mL, about 2 µg/mL, about 5 µg/mL, about 10 µg/mL, about 20 µg/mL, about 50 µg/mL, about 100 µg/mL, about 200 µg/mL, about 250 µg/mL, or about 500 µg/mL, about 1000 µg/mL, or in a range between these values, or a concentration that is less than these values.

In some embodiments, treatment is continued for a period of about 1 week or longer. In some embodiments, treatment is continued for a period of about 2 weeks or longer. In some embodiments, treatment is continued for a period of about 3 weeks or longer. In some embodiments, treatment is continued for a period of about 4 weeks or longer. In some embodiments, treatment is continued for a period of about 8 weeks or longer. In some embodiments, treatment is continued for a period of about 12 weeks, or about 13 weeks, or longer. In some embodiments, treatment is continued for a period of about 24 weeks, or 26 weeks, or longer. In some embodiments, treatment is continued for a period of about 6 months or longer. In some embodiments, treatment is continued for a period of about 12 months or longer. In some embodiments, treatment is continued for a period of about 18 months or longer. In some embodiments, treatment is continued for a period of about 24 months or longer.

### III. T-type Calcium Channel Antagonists

The T-type calcium channel antagonist according to the present invention is the compound MK-8998. Any other compounds are described herein for reference only.

The T-type calcium channel antagonist can be an antagonist of human T-type calcium channels when the subject of treatment is a human.

The T-type calcium channel antagonist according to the present disclosure can be a small molecule. Example small molecule T-type calcium channel antagonists which may be used in the methods provided herein include, but are not limited to, mibefradil, diltiazem, nifedipine, nitrendipine, nimodipine, niludipine, niguldipine, nicardipine, nisoldipine, amlodipine, felodipine, isradipine, ryosidine, gallopamil, verapamil, tiapamil, pimozide, thioridazine, NNC 55-0396, TTL-1177, anandamide, benzazepine derivatives, diphenylbutylpiperidine derivatives (e.g., pimozide, penfluridol, clopimozide, and fluspirilene), butyrophenone derivatives (e.g., haloperidol, droperidol, benperidol, triperidol, melperone, lenperone, azaperone, and domperidone), and phenylpiperazine derivatives (*e*.*g*., antrafenine, aripiprazole, ciprofloxacin, dapiprazole, dropropizine, etoperidone, itraconazole, ketoconazole, levodropropizine, mepiprazole, naftopidil, nefazodone, niaprazine, oxypertine, posaconazole, trazodone, urpidil, and vesnarinone), dihydropyridine derivatives (*e*.*g*., manidipine, nilvadipine, benidipine, and efonidipine), flunarizine, anandamide, lomerizine, zonisamide, U-92032, tetralol, tetralol derivatives *(e.g.,* mibefradil), mibefradil derivatives *(e.g.,* NNC 55-0396 dihydrochloride), TTA-A2, TTA-A8, TTA-P1, 4-aminomethyl-4-fluoropiperidine (TTA-P2), TTA-Q3, TTA-Q6, MIC-5395 (CX-5395), MK-6526, MK-8998 (CX-8998), Z941, Z944, succinimide anticonvulsant derivatives (*e.g.*, ethosuximide, phensuximide, and mesuximide also known as methsuximide, N-desmethylmethsuximide also known as (alpha)-methyl-(alpha)-phenyl-succinimide), and efonidipine (*e.g*. (R)-efonidipine), trimethadione, dimethadione, ABT-639, TTL-1177, KYSO5044, kurtoxin. Any of the T-type calcium channel inhibitors can be in the form of a pharmaceutically acceptable salt. Structures of representative T-type calcium channel inhibitors are shown below.

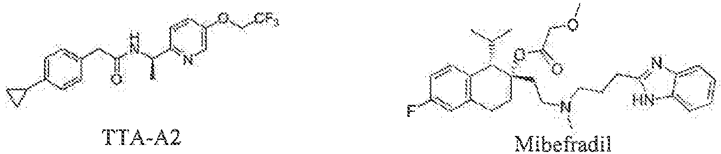

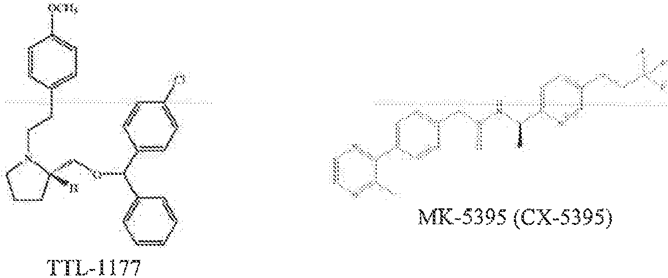

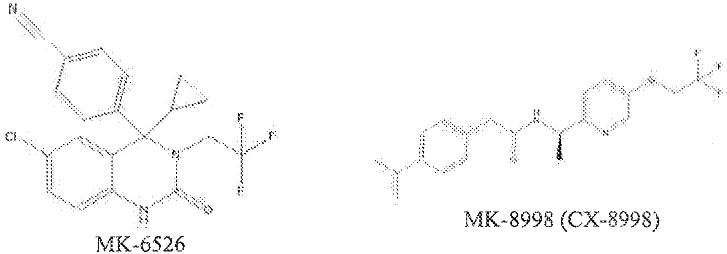

In some Z944 aspects of the disclosure, T-type calcium channel small-molecule antagonist may be selected from the group consisting of those described in the patents and published patent applications listed in Giordanetto et al, "T-type calcium channels inhibitors: a patent review," Expert Opin. Ther. Pat., 2011, 21, 85-101, including WO2004035000, WO9304047, WO2006098969, WO2009009015, WO2007002361, WO2007002884, WO2007120729, WO2009054982, WO2009054983, WO2009054984, US20090270413, WO2008110008, WO2009146539, WO2009146540, US8,133,998, WO2010083264, WO2006023881, WO2006023883, WO2005007124, WO2005009392, US2005245535, WO2007073497, WO200707852, WO2008033447, WO2008033456, WO2008033460, WO2008033464, WO2008033465, WO2008050200, WO20081 17148, WO2009056934, EP1568695, WO2008007835, KR754325, US7319098, US20100004286, EP1757590, KR2009044924, US2010094006, WO2009035307, US20090325979, KR75758317, WO2008018655, US20080293786, and US201000056545.

In some aspects of the disclosure the T-type calcium channel antagonist is a small molecule. In some aspects of the disclosure the small molecule has a molecular weight of 1000 or lower, e.g., about 900 or lower, about 800 or lower, about 700 or lower, about 600 or lower, about 500 or lower, about 400 or lower, or in the range from about 100 to about 500, about 200 to about 500, about 200 to about 400, about 300 to about 400 or about 300 to about 500.

In some aspects of the disclosure the T-type calcium channel antagonist is a selective T-type calcium channel antagonist. "Selective" in this context means that the T-type calcium channel antagonist is more potent at antagonizing T-type calcium channel calcium channels compared with other types of calcium channel, e.g., any one or more of L-type, N-type, P-type, Q-type and/or R-type calcium channels, e.g., compared with L-type calcium channels. Selectivity can be determined, e.g., by comparing the IC₅₀ of a compound in inhibiting T-type calcium channels with its IC₅₀ in inhibiting the other types of calcium channel: if the IC₅₀ for inhibiting T-type channels is lower than the IC₅₀ for inhibiting the other types of calcium channel, the compound is considered selective. An IC₅₀ ratio of 0.1 (or lower) denotes 10-fold (or greater) selectivity. An IC₅₀ ratio of 0.01 (or lower) denotes 100-fold (or greater) selectivity. An IC₅₀ ratio of 0.001 (or lower) denotes 1000-fold (or greater) selectivity. In some embodiments, the T-type calcium channel antagonist has selectivity for the T-type calcium channel that is 10-fold or greater, 100-fold or greater, or 1000-fold or greater compared with other types of calcium channel, e.g., any one or more of L-type, N-type, P-type, Q-type and/or R-type calcium channels, e.g., compared with L-type calcium channels.

In some aspects of the disclosure the T-type calcium channel antagonist is a selective T- type calcium channel inhibitor which is selected from the group consisting of phensuximide, methsuximide, methyl- phenyl-succinimide, R isomer of efonidipine, trimethadione, dimethadione, mibefradil, TTA-A2, TTA-A8, TTA-P1, TTA-P2, TTA-Q3, TTA-Q6, MK-5395 (CX-5395), MK-6526, MK-8998 (CX-8998), Z941, Z944, ABT-639, TTL-1177, KYS45444, N C 55-0396 dihydrochloride, kurtoxin, or a derivative thereof.

In some embodiments, the T-type calcium channel antagonist is a calcium channel antagonist that selectively targets T-type calcium channels over L-type calcium channels.

According to the invention, the T-type calcium channel antagonist is MK-8998.

In some embodiments, the treatment can be carried out without administration of verapamil. In some embodiments, the T-type calcium antagonist is administered in combination with verapamil.

In some embodiments, the treatment can be carried out without administration of ethosuximide. In some embodiments, the T-type calcium antagonist is administered in combination with ethosuximide.

In some embodiments, the treatment can be carried out without administration of zonisamide. In some embodiments, the T-type calcium antagonist is administered in combination with zonisamide.

In some embodiments, treatment can be carried out without administration of dimethadione. In some embodiments, the T-type calcium antagonist is administered in combination with dimethadione.

In some embodiments, the treatment can be carried out without administration of valproate. In some embodiments, the T-type calcium antagonist is administered in combination with valproate.

In some embodiments, the treatment can be carried out without administration of topiramate. In some embodiments, the T-type calcium antagonist is administered in combination with topiramate.

In some embodiments, the treatment can be carried out without administration of a cannabinoid such as cannabidiol or tetrahydrocannabinol. In some embodiments, the T-type calcium antagonist is administered in combination with a cannabinoid such as cannabidiol or tetrahydrocannabinol.

In some embodiments, the T-type calcium channel antagonist can be a molecule that does not act as a pore-blocker of the T-type calcium channel. The T-type calcium channel antagonist can be, e.g., an allosteric inhibitor of T-type calcium channels.

In some embodiments, the T-type calcium channel antagonist can be one that does not substantially affect one or more sodium channels such as sodium channels having Nav 1.1, Nav1.2, Nav1.3, Nav1.4, Nav1.5, Nav1.6, Nav1.7, Nav1.8, or Nav1.9 alpha subunits, and/or Navβ1, Navβ2, Navβ3, Navβ4 subunits. The T-type calcium channel antagonist can be selective for T-type calcium channel compared to inhibition of sodium channels, e.g., having at least a 2-fold, at least a 5-fold, at least a 10-fold, at least a 20-fold, at least a 100-fold, at least a 500-fold or at least a 1000-fold selectivity (expressed, *e.g.,* in terms of Kᵢ). The T-type calcium channel inhibitor can be one that does not substantially decrease the non-inactivating sodium current in thalamocortical neurons, e.g., that decreases the inactivating sodium current by about 20% or less, about 10% or less, about 5% or less, about 2% or less, or about 1% or less.

In some embodiments, the T-type calcium channel antagonist can be one that does not substantially affect one or more potassium channels such as calcium activated potassium channels (BK channels, SK channels, IK channels), inwardly rectifying potassium channels (ROMK, GPCR regulated, ATP sensitive), tandem pore domain potassium channels (TW1K (TWIK-1, TWIK-2, K.CNK7), TREK (TREK-1, TREIC-2, TRAAK), TASK (TASK-1, TASK-3, TASK-5), TALK (TASK-2, TALK-1, TALK-2), THIK (THIK-1, THIK-2), TRESK), or voltage gated potassium channels (hERG, KvLQT, KvLQT2). The T-type calcium channel antagonist can be selective for T-type calcium channel compared to inhibition of potassium channels, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ).

In some embodiments, the T-type calcium channel antagonist can be one that does not substantially affect one or more GABA receptors such as GABA_{A} receptors, GABA_{A-ρ} subclass (GABA_{C}) receptors, or GABA_{B} receptors. In some embodiments, the T-type calcium channel antagonist can be one that does not substantially affect one or more subunits of the GABA_{A} receptors such as α-subunits (GABRA1, GABRA2, GABRA3, GABRA4, GABRA5, GABRA6), β-subunits (GABRB1, GABRB2, GABRB3), γ-subunits (GABRG1, GABRG2, GABRG3), δ-subunits (GABRD), α-subunits (GABRE), α-subunits (GABRP), θ-subunits (GABRQ), particularly GABARA5, GABRB3 and GABRG5. The T-type calcium channel antagonist can be selective for T-type calcium channel compared to inhibition of GABA receptors, e.g., having at least a 2-fold, at least a 5-fold, at least a 10-fold, at least a 20-fold, at least a 100-fold, at least a 500-fold or at least a 1000-fold selectivity (expressed, *e.g.*, in terms of Kᵢ or binding affinity).

In some embodiments, the T-type calcium channel antagonist can be one that does not substantially affect one or more cannabinoid receptors such as cannabinoid receptor type 1 (CB1) or cannabinoid receptor type 2 (CB2) receptors. In some embodiments, the T-type calcium channel antagonist can be one that does not substantially affect CB1 receptors. In some embodiments, the T-type calcium channel antagonist can be one that does not substantially affect CB2 receptors. The T-type calcium channel antagonist can be selective for T-type calcium channel compared to inhibition of CB1 and/or CB2 receptors, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity).

In some embodiments, the T-type calcium channel antagonist can be one that does not substantially affect brain levels (e.g., CNS levels) of GABA. The T-type calcium channel antagonist can be selective for T-type calcium channel compared to increasing concentrations of GABA, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity, compared with the effective dose ED₅₀ for increasing the concentration of GABA).

In some embodiments, the T-type calcium channel antagonist can be one that does not substantially affect one or more AMPA or kainate glutamate receptors such as AMPA receptors comprising GluR1, GluR2, GluR3 or GluR4, e.g., combining two GluR2 units with two GluR1, two GluR3 or two GluR4 units and/or kainate receptors comprising GluR5, GluR6, GluR7, KA1 and/or KA2 receptors. In some embodiments, the T-type calcium channel antagonist can be one that does not substantially affect one or more subunits of the AMPA and/or kainite receptors such as those listed above. The T-type calcium channel antagonist can be selective for T-type calcium channel compared to inhibition of AMPA or kainate receptors, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity).

In some embodiments, the T-type calcium channel antagonist can be one that does not substantially inhibit histone deacetylase. The T-type calcium channel antagonist can be selective for T-type calcium channel compared to inhibition of histone deacetylase, *e.g.*, haying at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity compared with the IC₅₀ for inhibition of histone deacetylase).

In some embodiments, the T-type calcium channel antagonist can be one that does not substantially inhibit GABA transaminase. The T-type calcium channel antagonist can be selective for T-type calcium channel compared to inhibition of GABA transaminase, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity compared with the IC₅₀ for inhibition of GABA transaminase).

In some embodiments, the T-type calcium channel antagonist can be one that does not substantially inhibit succinate-semialdehyde dehydrogenase. The T-type calcium channel antagonist can be selective for T-type calcium channel compared to inhibition of succinate-semialdehyde dehydrogenase, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity compared with the IC₅₀ for inhibition of succinate-semialdehyde dehydrogenase).

In some embodiments, the T-type calcium channel antagonist can be one that does not substantially inhibit carbonic anhydrase, or one or more isozymes thereof. The T-type calcium channel antagonist can be selective for T-type calcium channel compared to inhibition of carbonic anhydrase, e.g., having at least a 2-fold, at least a 5-fold, at least a 10-fold, at least a 20-fold, at least a 100-fold, at least a 500-fold or at least a 1000-fold selectivity (expressed, e.g., in terms of Kᵢ or binding affinity compared with the IC₅₀ for inhibition of carbonic anhydrase).

In some embodiments, the T-type calcium channel antagonist can be one that does not cause one or more of the following side-effects or adverse events upon administration to animals, e.g., humans: liver damage, morphological changes in the animal liver, functional changes in the animal liver, kidney damage, morphological changes in the animal kidney, functional changes in the animal kidney, systemic lupus erythematosus, suicidal thoughts, suicidal behavior, suicidal ideation, increased risk of suicide, emergence or worsening of depression, unusual changes in mood or behavior, birth defects, allergic reaction.

In some embodiments, the T-type calcium channel antagonist can be one that does not cause one or more of the following side-effects or adverse events upon administration to animals: adverse events involving the gastrointestinal system such as anorexia, vague gastric upset, nausea and vomiting, cramps, epigastric and abdominal pain, weight loss, diarrhea, gum hypertrophy and swelling of the tongue; adverse events involving the hemopoietic system such as leukopenia, agranulocytosis, pancytopenia, with or without bone marrow suppression, and eosinophilia; adverse events involving the nervous system, including neurological reactions, sensory reactions, or psychiatric or psychological aberrations such as drowsiness, headache, dizziness, euphoria, hiccups, irritability, hyperactivity, lethargy, fatigue, ataxia, confusion, disturbances of sleep, night terrors, inability to concentrate, aggressiveness, paranoid psychosis, increased libido, or increased state of depression with overt suicidal intentions; adverse events involving the integumentary system including dermatologic manifestations such as urticaria, Stevens-Johnson syndrome, systemic lupus erythematosus, pruritic erythematous rashes, and hirsutism; adverse events involving the special senses such as myopia; and adverse events involving the genitourinary system, such as vaginal bleeding or microscopic hematuria.

In some aspects of the disclosure the T-type calcium channel antagonist is an antibody. Various methods for the preparation of antibodies are known in the art. See, Antibodies: A Laboratory Manual, CSH Press, Eds., Harlow, and Lane (1988); Harlow, Antibodies, Cold Spring Harbor Press, NY (1989). For example, antibodies can be prepared by immunizing a suitable mammalian host with a sample of whole cells isolated from a patient. Antibodies can be produced by cell culture techniques, including the generation of monoclonal antibodies as described herein, or *via* transfection of antibody genes into suitable bacterial or mammalian cell hosts, in order to allow for the production of recombinant antibodies.

In some aspects of the disclosure the antibody is a monoclonal antibody. A "monoclonal antibody" is an antibody obtained from a population of substantially homogeneous antibodies, *i.e.*, the antibodies comprising the population are identical except for possible naturally occurring mutations that are present in minor amounts.

In some aspects of the disclosure an antibody provided herein can be produced by recombinant means. In some aspects of the disclosure the antibody is a "humanized" or human antibody. "Humanized" or human antibodies can also be produced, and are preferred for use in therapeutic contexts. Methods for humanizing murine and other non-human antibodies, by substituting one or more of the non-human antibody sequences for corresponding human antibody sequences, are well known. See, *e.g.,* Jones et al., Nature, 1986, 321, 522-25; Riechmann et al., Nature, 1988, 332, 323-27; Verhoeyen et al., Science, 1988, 239, 1534-36, Carter et al., Proc. Natl. Acad. Sci. USA , 1993, 89. 4285; and Sims et al., J.Immunol., 1993, 151, 2296. These humanized antibodies are designed to minimize unwanted immunological response toward rodent antihuman antibody molecules which limits the duration and effectiveness of therapeutic applications of those moieties in human recipients. Accordingly, preferred antibodies according to the present disclosure are those that are either fully human or humanized with high affinity but exhibit low or no antigenicity in the subject.

In some aspects of the disclosure the T-type calcium channel antagonist is an oligonucleotide inhibitor. Example oligonucleotide inhibitors include, but are not limited to, antisense oligonucleotides, RNAi, dsRNA, siRNA and ribozymes. In some embodiments, the T-type calcium channel antagonist is a siRNA. As used in the specification, "antisense oligonucleotide" refers to a stretch of single-stranded DNA or RNA, usually chemically modified, whose sequence (3'-5') is complementary to the sense sequence of a molecule of mRNA. Antisense molecules effectively inhibit gene expression by forming RNA/DNA duplexes. Antisense is understood to work by a variety of mechanisms, including physically blocking the ability of ribosomes to move along the messenger RNA, and hastening the rate at which the mRNA is degraded within the cytosol.

In order to avoid digestion by DNAse, antisense oligonucleotides can be chemically modified. For example, phosphorothioate oligodeoxynucleotides are stabilized to resist nuclease digestion by substituting one of the non-bridging phosphoryl oxygen of DNA with a sulfur moiety. Increased antisense oligonucleotide stability can also be achieved using molecules with 2-methoxyethyl (MOE) substituted backbones as described generally in U.S. Pat. No. 6,451,991, incorporated by reference, and U.S. Pat. Appl. Publ. No. 2003/0158143-A1. Thus, the antisense oligonucleotide can be modified to enhance in vivo stability relative to an unmodified oligonucleotide of the same sequence. The modification may be, e.g., a (2'-**O-2-methoxyethyl) modification.** The oligonucleotide may have a phosphorothioate backbone throughout, the sugar moieties of nucleotides 1-4 and 18-21 may bear 2'-O-methoxyethyl modifications and the remaining nucleotides may be 2'-deoxynucleotides.

It is understood in the art that an antisense oligonucleotide need not have 100% identity with the complement of its target sequence in order to be effective. The antisense oligonucleotides, therefore, can have a sequence that is at least about 70% identical to the complement of the target sequence. In one embodiment, the antisense oligonucleotides have can a sequence that is at least about 80% identical to the complement of the target sequence. In other embodiments, they have a sequence that is at least about 90% identical or at least about 95%. identical to the complement of the target sequence, allowing for gaps or mismatches of several bases. Identity can be determined, for example, by using the BLASTN program of the University of Wisconsin Computer Group (GCG) software.

The antisense oligonucleotides according to the present disclosure are typically between 7 and 100 nucleotides in length. In one aspect of the disclosure the antisense oligonucleotides comprise from about 7 to about 50 nucleotides, or nucleotide analogues. In another aspect of the disclosure the antisense oligonucleotides comprise from about 7 to about 35 nucleotides, or nucleotide analogues. In other aspects of the disclosure the antisense oligonucleotides comprise from about 12 to about 35 nucleotides, or nucleotide analogues, and from about 15 to about 25 nucleotides, or nucleotide analogues.

The oligonucleotide inhibitors according to the present disclosure can be siRNA molecules that are targeted to a gene of interest such that the sequence of the siRNA corresponds to a portion of said gene. RNA molecules according to the present disclosure generally comprise an RNA portion and some additional portion, for example a deoxyribonucleotide portion.

The present disclosure further contemplates ribozyme oligonucleotide modulators that specifically target mRNA encoding a protein of interest, such as the proteins comprising the T-type calcium channel. Ribozymes are RNA molecules having an enzymatic activity that enables the ribozyme to repeatedly cleave other separate RNA molecules in a nucleotide-sequence specific manner. Such enzymatic RNA molecules can be targeted to virtually any mRNA transcript, and efficient cleavage can be achieved in vitro. Kim et al., Proc. Natl. Acad. Sci. USA, 1987, 84, 8788; Haseloff et al., Nature, 1988, 334, 585; Cech, JAMA, 1988, 260, 3030; and Jefferies et al., Nucleic Acids Res., 1989, 17, 1371.

Typically, a ribozyme comprises two portions held in close proximity: an mRNA binding portion having a sequence complementary to the target mRNA sequence and a catalytic portion which acts to cleave the target mRNA. A ribozyme acts by first recognizing and binding a target mRNA by complementary base-pairing through the target mRNA binding portion of the ribozyme. Once it is specifically bound to its target, the ribozyme catalyzes cleavage of the target mRNA. Such strategic cleavage destroys the ability of a target mRNA to direct synthesis of an encoded protein. Having bound and cleaved its mRNA target, the ribozyme is released and can repeatedly bind and cleave new target mRNA molecules.

In some embodiments, the selective T-type calcium channel antagonist substantially crosses the blood brain barrier.

In some embodiments, the selective T-type calcium channel antagonist does not substantially cross the blood brain barrier.

In some embodiments, the T-type calcium channel antagonist is a calcium channel antagonist that selectively targets T-type calcium channels. In some embodiments, the T-type calcium channel antagonist is a small molecule as described herein.

In some embodiments, the T-type calcium channel antagonist selectively targets Cav3.1. In some embodiments, the T-type calcium channel antagonist selectively targets Cav3.2. In some embodiments, the T-type calcium channel antagonist selectively targets Cav3.3. "Selective" in this context means that the T-type calcium channel antagonist is more potent at antagonizing one type of T-type calcium channel over another type of calcium channel, e.g., more potent at antagonizing Cav3.1 than Cav3.2 or Cav3.3 or both; more potent at antagonizing Cav3.2 than Cav3.1 or Cav3.3 or both; more potent at antagonizing Cav3.3 than Cav3.1 or Cav3.2 or both. Selectivity can be determined, e.g., by comparing the IC₅₀ of a compound in inhibiting one type of T-type calcium channel with its IC₅₀ in inhibiting the other types of T-type calcium channel: if the IC₅₀ for inhibiting one type of T-type channels is lower than the IC₅₀ for inhibiting the other type of T-type calcium channel, the compound is considered selective. An IC₅₀ ratio of 0.1 (or lower) denotes 10-fold (or greater) selectivity. An IC₅₀ ratio of 0.01 (or lower) denotes 100-fold (or greater) selectivity. An IC₅₀ ratio of 0.001 (or lower) denotes 1000-fold (or greater) selectivity. In some embodiments, the selectivity for Cav3.1, Cav3.2 or Cav3.3 is 10-fold or greater, 100-fold or greater, or 1000-fold or greater.

In some embodiments, the T-type calcium channel antagonist selectively targets T-type calcium channels (e.g., Cav3.1, Cav3.2, and/or Cav3.3) over sodium channels such as sodium channels having Nav 1.1, Nav 1.2, Nav 1.3, Nav 1.4, Nav 1.5, Nav 1.6, Nav 1.7, Nav 1.8, or Nav 1.9 alpha subunits, and/or Nav β1, Nav β2, Nav β3, Nav β4 subunits. The T-type calcium channel antagonist can be selective for T-type calcium channel compared to inhibition of sodium channels. Selectivity can be determined, e.g., by comparing the IC₅₀ of a compound in inhibiting one or more of the types of T-type calcium channel with its IC₅₀ in inhibiting the one or more types of sodium channel: if the IC₅₀ for inhibiting the T-type calcium channels is lower than the IC₅₀ for inhibiting the sodium channel, the compound is considered selective. An IC₅₀ ratio of 0.1 (or lower) denotes 10-fold (or greater) selectivity. An IC₅₀ ratio of 0.01 (or lower) denotes 100-fold (or greater) selectivity. An IC₅₀ ratio of 0.001 (or lower) denotes 1000-fold (or greater) selectivity. In some embodiments, the selectivity for T-type calcium channels is 10-fold or greater, 100-fold or greater, or 1000-fold or greater.

The effectiveness of a compound in inhibiting T-type calcium channels may vary depending on the state of the T-type calcium channel that the T-type calcium channel antagonist inhibits. T-type calcium channels can occur in different states depending on the cell membrane potential. T-type calcium channel antagonists that are effective in the methods described herein may include T-type calcium channel antagonists that block T-type calcium channels when the membrane potential is in the range from about -60 mV to about -30 mV, e.g., preferably about -40 mV. A membrane potential "in the range from about -60 to about -30 mV" can include membrane potentials within a range of -70 mV to -20 mV, or within a range of -65 mV to -25 mV, and can also encompass membrane potential ranges such as about -40 mV to about -30 mV, about -50 mV to about -30 mV, about -70 mV to about -30 mV, about -50 mV to about -40 mV, about -60 mV to about -40 mV, about -70 mV to about -40 mV, about -60 mV to about -50 mV, and about -70 to about -50 mV, as well as about -30 mV, about -40 mV, about -50 mV, and about -60 mV. In some embodiments, the T-type calcium channel antagonists that are effective in the methods described herein may include T-type calcium channel antagonists that block T-type calcium channels when the membrane potential is in the range from about -100 mV to about -80 mV, e.g., preferably about -90 mV. A membrane potential "in the range from about -100 to about -80 mV" can include membrane potentials within a range of -110 mV to -70 mV, or within a range of -105 mV to -75 mV, and can also encompass membrane potential ranges such as about -100 mV to about -80 mV, about -90 mV to about -80 mV, and about -100 mV to about -90 mV, as well as about -100 mV, about -90 mV, and about -80 mV.

While not being limited by any theory, it is believed that T-type calcium channel antagonists that are effective in the methods described herein may include T-type calcium channel antagonists that block T-type calcium channels when the membrane potential is in the range from about -60 mV to about -30 mV, e.g., about -40 mV selectively when compared to blockade of the T-type calcium channels when the membrane potential is in the range from about -100 mV to about -80 mV, e.g., about -90 mV.

A T-type channel inhibitor that is effective may inhibit T-type calcium channels with an IC₅₀ for inhibiting T-type calcium channels when the membrane potential is about -40 mV that is about 10 µM or lower, e.g., about 1 µM or lower, about 500 nM or lower, about 100 nM or lower, about 50 nM or lower, about 10 nM or lower, about 5 nM or lower, or about 1 nM or lower. A T-type calcium channel antagonist that is effective may inhibit T-type calcium channels at a membrane potential of about -40 mV selectively compared to inhibition of T-type calcium channels at a membrane potential of about -90 mV. For example, the ratio of the IC₅₀ of the T-type calcium channel antagonist in inhibiting T-type calcium channels at a membrane potential of about -40 mV selectively compared to inhibition of T-type calcium channels at a membrane potential of about -90 mV may be about 1:2 or lower, *e.g*., about 1:5 or lower, about 1:10 or lower, about 1:20 or lower, about 1:50 or lower, about 1:100 or lower, about 1:500 or lower, about 1:1000 or lower. In some embodiments, the selectivity for inhibiting T-type calcium channels at about -40 mV compared to inhibiting T-type calcium channels at about -90 mV is 2-fold or greater, 5-fold or greater, 10-fold or greater, 100-fold or greater, or 1000-fold or greater.

A T-type channel inhibitor that is effective may inhibit T-type calcium channels with an IC₅₀ for inhibiting T-type calcium channels when the membrane potential is about -90 mV that is about 10 µM or lower, e.g., about 1 µM or lower, about 500 nM or lower, about 100 nM or lower, about 50 nM or lower, about 10 nM or lower, about 5 nM or lower, or about 1 nM or lower. A T-type calcium channel antagonist that is effective may inhibit T-type calcium channels at a membrane potential of about -90 mV selectively compared to inhibition of T-type calcium channels at a membrane potential of about -40 mV. For example, the ratio of the IC₅₀ of the T-type calcium channel antagonist in inhibiting T-type calcium channels at a membrane potential of about -90 mV selectively compared to inhibition of T-type calcium channels at a membrane potential of about -40 mV may be about 1:2 or lower, e.g., about 1:5 or lower, about 1:10 or lower, about 1:20 or lower, about 1:50 or lower, about 1:100 or lower, about 1:500 or lower, about 1:1000 or lower. In some embodiments, the selectivity for inhibiting T-type calcium channels at about -90 mV compared to inhibiting T-type calcium channels at about -40 mV is 2-fold or greater, 5-fold or greater, 10-fold or greater, 100-fold or greater, or 1000-fold or greater.

All compounds, and pharmaceutically acceptable salts thereof, can be found together with other substances such as water and solvents (e.g. hydrates and solvates) or can be isolated. In some embodiments, the compounds provided herein, or pharmaceutically acceptable salts thereof, are substantially isolated. By "substantially isolated" is meant that the compound is at least partially or substantially separated from the environment in which it was formed or detected. Partial separation can include, for example, a composition enriched in the compounds provided herein. Substantial separation can include compositions containing at least about 50%, at least about 60%, at least about 70%, at least about 80%, at least about 90%, at least about 95%, at least about 97%, or at least about 99% by weight of the compounds provided herein, or salt thereof. Methods for isolating compounds and their salts are routine in the art.

As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present application include the conventional non-toxic salts of the parent compound formed, for example, from non-toxic inorganic or organic acids. The pharmaceutically acceptable salts of the present application can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (e.g., methanol, ethanol, iso-propanol, or butanol) or acetonitrile are preferred. Lists of suitable salts are found in Remington's Pharmaceutical Sciences, 17th ed., Mack Publishing Company, Easton, Pa., 1985, p. 1418 and Journal of Pharmaceutical Science, 66, 2 (1977). Methods for preparing salt forms are described, for example, in Handbook of Pharmaceutical Salts: Properties, Selection, and Use, Wiley-VCH, 2002.

### IV. Combination Therapies

One or more additional therapeutic agents can be used in combination with the compounds provided herein for the treatment of Dravet syndrome. Example additional therapeutic agents include, but are not limited to calcium channel antagonists (including L-type and T-type calcium channel antagonists), anticonvulsant agents, GABA(A) receptor agonists, and positive allosteric modulators or gene therapy or gene reactivation therapy.

In some embodiments, the treatment with the T-type calcium channel antagonist can be provided in the absence of additional therapeutic agents useful for treating Dravet syndrome. In some embodiments, the treatment can be performed with a single T-type calcium channel antagonist. In some embodiments, the treatment with the T-type calcium channel antagonist can be provided in the absence of an anticonvulsant agent.

The one or more additional therapeuyic agents can be administered to a patient simultaneously or sequentially, using the same schedule or a different schedule of administration, which will be determined by the particular combination used and the judgment of the prescribing physician.

Example calcium channel antagonists include, but are not limited to, the T-type calcium channel antagonists described herein, and L-type calcium channel antagonists. In some embodiments, the additional calcium channel antagonist is selected from a T-type calcium channel antagonist provided herein. In some embodiments, the additional calcium channel antagonist is an L-type calcium channel antagonist. In some embodiments, the additional calcium channel antagonist is a T-type calcium channel antagonist. In some embodiments, the additional calcium channel antagonist is a T-type calcium channel antagonist selected from the group consisting of mibefradil, MK-5395 (CX-5395), MK-6526, MK-8998 (CX-8998), and Z944. In some embodiments, the additional calcium channel antagonist is a T-type calcium channel antagonist and an L-type calcium channel antagonist. In some embodiments, the additional calcium channel antagonist is a T-type calcium channel antagonist or an L-type calcium channel antagonist selected from the group consisting of ACT-28077, mibefradil, and TTL-1177. In some embodiments, the additional calcium channel antagonist is mibefradil.

Example anticonvulsant agents include, but are not limited to, acetazolamide, clobazam, clonazepam, eslicarbazepine acetate, ethosuximide, lacosamide, levetiracetam, nitrazepam, oxcarbazepine, perampanel, piracetam, phenobarbital, pregabalin, primidone, retigabine, rufinamide, valproate, *e*.*g*., sodium valproate, stiripentol, tiagabine, topiramate, vigabatrin, and zonisamide.

Example GABA(A) receptor agonists include gaboxadol, bamaluzole, gamma-aminobutyric acid, gabamide, gamma-amino-beta-hydroxybutyric acid, gaboxadol, ibotenic acid, isoguvacine, isonipecotic acid, muscimol, phenibut, picamilon, progabide, quisqualamine, SL 75102, and thiomuscimol.

Example GABA(A) receptor positive allosteric modulators include avermectins (*e*.*g*., ivermectin), barbiturates (e.g., phenobarbital), benzodiazepines (*e*.*g*., adinazolam, alprazolam, bentazepam, bretazenil, bromazepam, brotizolam, camazepam, chlordiazepoxide, cinazepam, cinolazepam, clobazam, clonazepam, clonazolam, clorazepate, clotiazepam, cloxazolam, delorazepam, diazepam, diclazepam, estazolam, ethyl carfluzepate, etizolam, ethyl loflazepate, flubromazepam, flubromazolam, flunitrazepam, flurazepam, flutazolam, ethyl loflazepate, flubromazepam, flubromazolam, flunitrazepam, flurazepam, flutazolam, flutoprazepam, halazepam, ketazolam, loprazolam, lorazepam, lormetazepam, medazepam, mexazolam, midazolam, nifoxipam, nimetazepam, nitrazepam, nordiazepam, oxazepam, phenazepam, pinazepam, prazepam, premazepam, pyrazolam, quazepam, rilmazafone, temazepam, thienalprazolam, tetrazepam, and triazolam), bromides (*e*.*g*., potassium bromide, carbamates (*e*.*g*., meprobamate, carisoprodol), chloralose, chlormezanone, clomethiazole, dihydroergolines (*e*.*g*., ergoloid (dihydroergotoxine)), etazepine, etifoxine, Imidazoles (*e*.*g*., etomidate), kavalactones (found in kava), loreclezole, neuroactive steroids (*e*.*g*., allopregnanolone, ganaxolone), nonbenzodiazepines (*e*.*g*., zaleplon, zolpidem, zopiclone, eszopiclone), petrichloral, phenols (*e*.*g*., propofol), piperidinediones (e.g., glutethimide, methyprylon), propanidid, pyrazolopyridines (*e*.*g*., etazolate), quinazolinones (e.g., methaqualone), skullcap constituents, stiripentol, sulfonylalkanes (*e*.*g*., sulfonmethane, tetronal, trional), and valerian constituents (*e*.*g*., valeric acid, valerenic acid).

In some embodiments, the therapy can be administered as a monotherapy. In some embodiments, the therapy can be administered in the absence of additional antiepileptic therapy. The therapy can be administered in the absence of any of the additional agents described in this section. For example, the therapy can be administered in the absence of additional anticonvulsant such as, acetazolamide, clobazam, clonazepam, eslicarbazepine acetate, ethosuximide, lacosamide, levetiracetam, nitrazepam, oxcarbazepine, perampanel, piracetam, phenobarbital, pregabalin, primidone, retigabine, rufinamide, valproate, *e.g.*, sodium valproate, stiripentol, tiagabine, topiramate, vigabatrin, or zonisamide.

In some embodiments, the T-type calcium channel antagonists provided herein can be used in combination with one or more additional therapies including, but not limited to, a ketogenic diet, physical therapy, occupational therapy, communication therapy (e.g., speech therapy), and behavioral therapy.

In some embodiments, the T-type calcium channel antagonists provided herein can be used in combination with one or more additional therapeutic agents and one or more additional therapies selected from the group consisting of a ketogenic diet, physical therapy, occupational therapy, communication therapy (e.g., speech therapy), and behavioral therapy.

### V. Pharmaceutical Compositions

The T-type calcium channel inhibitors used in the methods described herein can be administered in the form of pharmaceutical compositions. Thus the present disclosure provides T-type calcium channel inhibitor, and at least one pharmaceutically acceptable carrier for use in the claimed methods of treatment, or the manufacture of a medicament for treating conditions as described herein. These compositions can be prepared in a manner known in the pharmaceutical art, and can be administered by a variety of routes. Administration may be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or may be, *e.g.,* by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

This application provides pharmaceutical compositions which contain, as the active ingredient, a T-type calcium channel inhibitor (which can be in the form of a pharmaceutically acceptable salt), in combination with one or more pharmaceutically acceptable carriers (excipients). In some embodiments, the composition is suitable for topical administration. In making the compositions of the invention, the active ingredient is typically mixed with an excipient, diluted by an excipient or enclosed within such a carrier in the form of, *e.g.,* a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, e.g., up to 10% by weight of the active compound, soft and hard gelatin capsules, suppositories, sterile injectable solutions and sterile packaged powders.

In preparing a formulation, the T-type calcium channel inhibitor can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g., about 40 mesh.

The compounds of the invention may be milled using known milling procedures such as wet milling to obtain a particle size appropriate for tablet formation and for other formulation types. Finely divided (nanoparticulate) preparations of the compounds of the invention can be prepared by processes known in the art.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup and methyl cellulose. The Formulations can additionally include: lubricating agents such as talc, magnesium stearate and mineral oil; wetting agents; emulsifying and suspending agents; preserving agents such as methyl- and propylhydroxy-benzoates; sweetening agents; and flavoring agents.

In some embodiments, the pharmaceutical composition comprises silicified microcrystalline cellulose (SMCC) and at least one compound described herein, or a pharmaceutically acceptable salt thereof. In some embodiments, the silicified microcrystalline cellulose comprises about 98% microcrystalline cellulose and about 2% silicon dioxide wttwt.

In some embodiments, a wet granulation process is used to produce the composition. In some embodiments, a dry granulation process is used to produce the composition.

The compositions can be formulated in a unit dosage form, each dosage containing from about 5 to about 1,000 mg (1 g), more usually about 100 mg to about 500 mg, of the active ingredient. In some embodiments, each dosage contains about 10 mg of the active ingredient. In some embodiments, each dosage contains about 50 mg of the active ingredient. In some embodiments, each dosage contains about 25 mg of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The components used to formulate the pharmaceutical compositions are of high purity and are substantially free of potentially harmful contaminants (e.g., at least National Food grade, generally at least analytical grade, and more typically at least pharmaceutical grade). Particularly for human consumption, the composition is preferably manufactured or Formulated under Good Manufacturing Practice standards as defined in the applicable regulations of the U.S. Food and Drug Administration. For example, suitable Formulations may be sterile and/or substantially isotonic and/or in full compliance with all Good Manufacturing Practice regulations of the U.S. Food and Drug Administration.

The active compound may be effective over a wide dosage range and is generally administered in a therapeutically effective amount. It will be understood, however, that the amount of the compound actually administered will usually be determined by a physician, according to the relevant circumstances, including the condition to be treated, the chosen route of administration, the actual compound administered, the age, weight, and response of the individual patient, the severity of the patient's symptoms and the like.

The therapeutic dosage of a compound of the present invention can vary according to, *e.g.,* the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (e.g., hydrophobicity), and the route of administration. For example, the compounds of the invention can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables severity of the disease, the overall health status of the particular patient, the relative biological efficacy of the compound selected, Formulation of the excipient, and its route of administration. Effective doses can be extrapolated from dose-response curves derived from *in vitro* or animal model test systems. Effective doses for a human can be, e.g., about 1 mg, 2 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 250 mg, 300 mg, 350 mg, 400 mg, 500 mg, 600 mg, 700 mg, 800 mg, 900 mg or 1000 mg. The doses can be administered, e.g., once a day, twice a day, three times a day, or four times a day.

In some aspects of the disclosure, when the T-type calcium channel antagonist is mibefradil, and the mibefradil can be administered at a dose of, e.g., about 0.1 mg, 0.3 mg, 1 mg, 3 mg, 5 mg, 10 mg. 15 mg. or 30 mg. The doses can be administered, e.g., once a day, twice a day, three times a day, or four times a day.

In some aspects of the disclosure, when the T-type calcium channel antagonist is MK-5395 (CX-5395), and the MK-5395 can be administered at a dose of, e.g., about 0.3 mg/kg, 1 mg/kg, 3 mg/kg, 5 mg/kg, 10 mg/kg. 30 mg/kg, or 100 mg/kg. The doses can be administered, e.g., once a day, twice a day, three times a day, or four times a day.

In some aspects of the disclosure, the T-type calcium channel antagonist is MK-6526, and the MK-6526 can be administered at a dose of, e.g., about 0.3 mg/kg, 1 mg/kg, 3 mg/kg, 5 mg/kg, 10 mg/kg, 30 mg/kg, or 100 mg/kg. The doses can be administered, e.g., once a day, twice a day, three times a day, or four times a day.

According to the invention, the T-type calcium channel antagonist is MK-8998 (CX-8998), and the MK-8998 can be administered at a dose of, e.g., about 0.3 mg/kg, 1 mg/kg, 3 mg/kg, 5 mg/kg, 10 mg/kg. 30 mg/kg, or 100 mg/kg. The doses can be administered, e.g., once a day, twice a day, three times a day, or four times a day.

In some aspects of the disclosure, the T-type calcium channel antagonist is Z944, and the Z944 can be administered at a dose of, e.g., about 0.3 mg/kg, 1 mg/kg, 3 mg/kg, 5 mg/kg, 10 mg/kg. 30 mg/kg, or 100 mg/kg. The doses can be administered, e.g., once a day, twice a day, three times a day, or four times a day.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, e.g., about 0.1 to about 1000 mg of the active ingredient of the present invention.

The liquid forms in which the compounds and compositions of the present invention can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face mask, tent, or intermittent positive pressure breathing machine. Solution, suspension, or powder compositions can be administered orally or nasally from devices which deliver the Formulation in an appropriate manner.

Topical formulations can contain one or more carriers. In some embodiments, ointments can contain water and one or more hydrophobic carriers selected from, e.g., liquid paraffin, polyoxyethylene alkyl ether, propylene glycol, white petroleum jelly, and the like. Carrier compositions of creams can be based on water in combination with glycerol and one or more other components, e.g., glycerinemonostearate, PEG-glycerinemonostearate and cetylstearyl alcohol. Gels can be formulated using isopropyl alcohol and water, suitably in combination with other components such as, e.g., glycerol, hydroxyethyl cellulose, and the like. In some embodiments, topical formulations contain at least about 0.1, at least about 0.25, at least about 0.5, at least about 1, at least about 2 or at least about 5 wt% of the compound of the invention.

The amount of compound or composition administered to a patient will vary depending upon what is being administered, the purpose of the administration, such as prophylaxis or therapy, the state of the patient, the manner of administration and the like. In therapeutic applications, compositions can be administered to a patient already suffering from a disease in an amount sufficient to eliminate or at least partially alleviate the symptoms of the disease and its complications. Effective doses will depend on the disease condition being treated as well as by the judgment of the attending clinician depending upon factors such as the severity of the disease, the age, weight and general condition of the patient and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers or stabilizers will result in the formation of pharmaceutical salts.

The therapeutic dosage of a T-type calcium channel antagonist used in the methods described herein can vary according to, e.g., the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (e.g., hydrophobicity), and the route of administration. For example, the T-type calcium channel antagonists can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or *in vivo* model test systems.

### EXAMPLES

The invention is further described in the following examples.

### Example 1. Effect of CACNA1G-KnockOnt on Dravet Phenotype

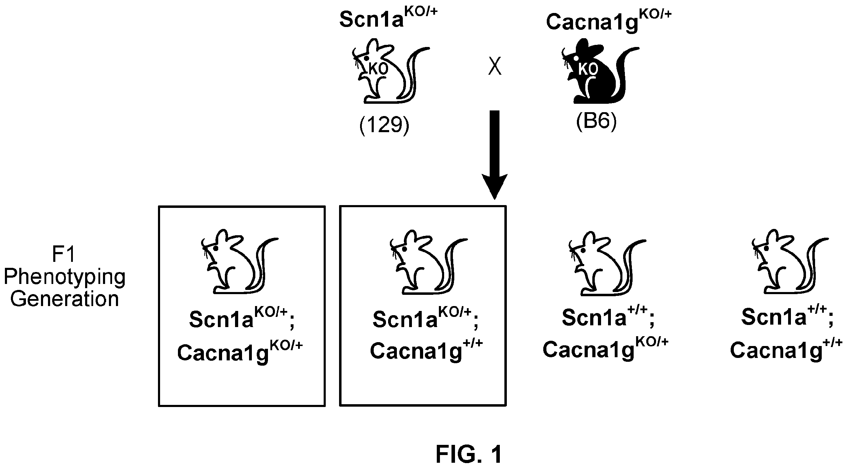

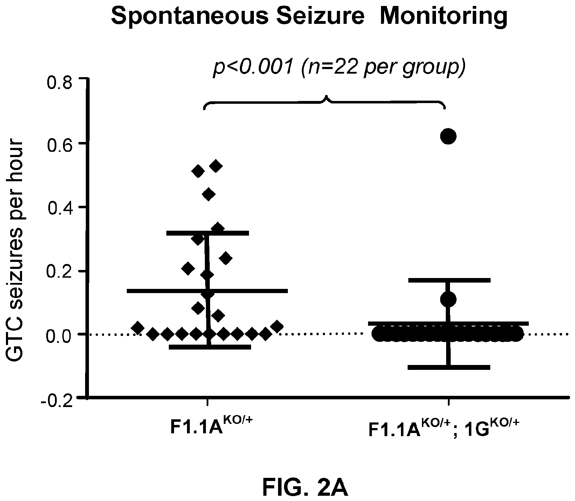

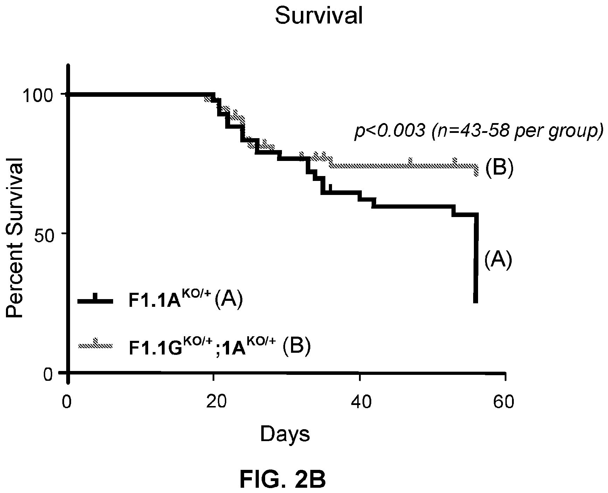

A Dravet mouse model (see *e.g.,* Miller et al, Genes Brain Behav. 2014, 13:163-72) was bred with a Cav3.1 genetic knockout (KO) mouse to produce heterozygous KO of Cacnalg in Dravet mouse model (FIG. 1). The Dravet mouse and Cav3.1 KO were tested for spontaneous tonic-clonic seizures as well as survival. As shown in FIG. 2A and FIG. 2B, heterozygous deletion of Cacnalg provided a protective benefit for spontaneous generalized tonic-clonic seizures and survival in the *Scn1a*^{+/-} Dravet model. These data indicate that Cav3.1 may be a therapeutic target useful in treating Dravet syndrome.

### Example 2. Effects of TTA-A2 on Scn1a^{+/-} mouse model

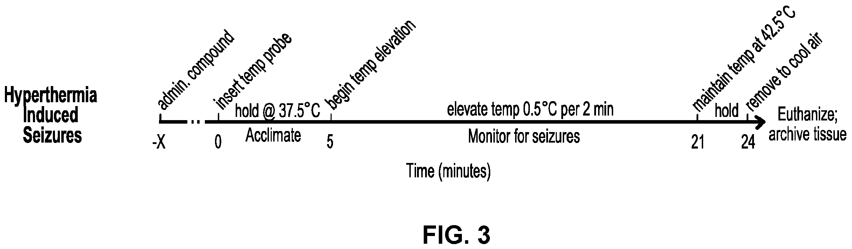

TTA-A2, a selective Cav3 antagonist, will be evaluated in a hyperthermia induced seizure screen in the *Scn1a*^{+/-} mouse model (see *e.g.,* Miller et al, Genes Brain Behav. 2014, 13:163-72). Mice will be randomly assigned treatment or control groups and will be administered TTA-A2 or vehicle by oral gayage. Hyperthermia will be induced until seizure occurs or maximal temp is reached, for example, as shown in FIG. 3. The number of generalized tonic-clonic (GTC) mice will be compared between control and treatment group. As shown in FIG. 3, "-X" minutes will be determined based on TTA-A2 pharmacokinetics such that peak brain exposure occurs during seizure induction. Pharmacokinetic data for TTA-A2 is shown below in Tables 2-3. Animal temperature will be elevated until 42.5° C and held for three minutes or until GTC seizure is observed.

**Table 2.**

| | |
|---|---|
| **α1l** | 9 nM |
| **α1l+Kir** | 296 nM |
| **α1l VC (-100mV)** | 4200 nM |
| **α1l VC (-80 mV)** | 98 nM |
| **hERG** | >10000 nM |
| **L-Type** | >10000 nM |
| **N-Type** | >10000 nM |
| **Na Channel** | >10000 nM |
| **Panlabs** | 0 hits >50%@10 uM |
| **PGP, Papp** | 0.7, 49 x 10⁻⁶ cm/s |
| **WAG/Rij (10mpk)** | 87% inhibition@4 hr |

**Table 3.**

| Species | IV (DMSO) | | PO (1% methyicellulose) | | | |
|---|---|---|---|---|---|---|
| | Clp mL/min/kg | T_{1/2} (hr) | Cₘₐₓ (uM) | F (%) | AUC (uM•hr) | Dose (mpk) |
| Rat | 6 | 1.5 | 7.5 | 68 | 46 | 10 |
| Dog | 0.9 | 11 | 2.1 | 43 | 23 | 1 |
| Monkey | 17 | 0.9 | 0.3 | 7 | 0.6 | 3 |

## Claims

1. A T-type calcium channel antagonist for use in a method of treating Dravet syndrome in a subject, wherein the method comprises administering to the subject in need thereof a therapeutically effective amount of the T-type calcium channel antagonist, wherein the T-type calcium channel antagonist is MK-8998: 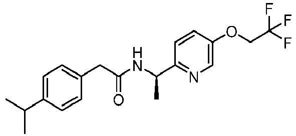 or a pharmaceutically acceptable salt thereof.

2. The T-type calcium channel antagonist for use of claim 1, wherein the treatment comprises reducing or ameliorating at least one neurological symptom in the subject.

3. The T-type calcium channel antagonist for use of claim 2, wherein the neurological symptom comprises one or more of seizure, hyperactivity, impulsiveness, autistic behavior, somnolence, insomnia, psychomotor delay, ataxia cognitive impairment, neurological development, developmental delay, and impaired behavior.

4. The T-type calcium channel antagonist for use of claim 3, wherein the neurological symptom comprises a seizure and the seizure is a febrile seizure.

5. The T-type calcium channel antagonist for use of any one of claims 1 to 4, wherein the treatment comprises improving the short-term memory or the working memory of the subject.

6. The T-type calcium channel antagonist for use of any one of claims 1 to 5, further comprising administering to the subject an additional therapeutic agent.

7. The T-type calcium channel antagonist for use of claim 6, wherein the additional therapeutic agent is an additional T-type calcium channel inhibitor.

8. The T-type calcium channel antagonist for use of claim 7, wherein the additional therapeutic agent is an anticonvulsive agent.

9. The T-type calcium channel antagonist for use of claim 8, wherein the anticonvulsive agent is selected from acetazolamide, clobazam, clonazepam, eslicarbazepine acetate, ethosuximide, lacosamide, levetiracetam, nitrazepam, oxcarbazepine, perampanel, piracetam, phenobarbital, pregabalin, primidone, retigabine, rufinamide, valproate, stiripentol, tiagabine, topiramate, vigabatrin, and zonisamide.

10. The T-type calcium channel antagonist for use of any one of claims 1 to 9, further comprising administering an additional therapy selected from the group consisting of a ketogenic diet, physical therapy, occupational therapy, communication therapy, and behavioral therapy.

## Patentansprüche

1. T-Typ-Calciumkanal-Antagonist zur Verwendung bei einem Verfahren zur Behandlung des Dravet-Syndroms bei einem Individuum, wobei das Verfahren das Verabreichen einer therapeutisch wirksamen Menge des T-Typ-Calciumkanal-Antagonisten an das Individuum, das dies benötigt, umfasst, wobei der T-Typ-Calciumkanal-Antagonist MK-8998: 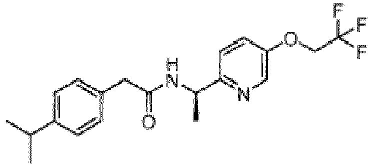 oder ein pharmazeutisch akzeptables Salz davon ist.

2. T-Typ-Calciumkanal-Antagonist zur Verwendung nach Anspruch 1, wobei die Behandlung das Verringern oder Lindern mindestens eines neurologischen Symptoms bei dem Individuum umfasst.

3. T-Typ-Calciumkanal-Antagonist zur Verwendung nach Anspruch 2, wobei das neurologische Symptom eines oder mehrere aus Krampfanfall, Hyperaktivität, Impulsivität, autistischem Verhalten, Somnolenz, Schlaflosigkeit, psychomotorischer Verzögerung, Ataxie kognitiver Beeinträchtigung, neurologischer Entwicklung, Entwicklungsverzögerung und beeinträchtigtem Verhalten umfasst.

4. T-Typ-Calciumkanal-Antagonist zur Verwendung nach Anspruch 3, wobei das neurologische Symptom einen Krampfanfall umfasst und der Krampfanfall ein Fieberkrampf ist.

5. T-Typ-Calciumkanal-Antagonist zur Verwendung nach einem der Ansprüche 1 bis 4, wobei die Behandlung das Verbessern des Kurzzeitgedächtnisses oder des Arbeitsgedächtnisses des Individuums umfasst.

6. T-Typ-Calciumkanal-Antagonist zur Verwendung nach einem der Ansprüche 1 bis 5, das ferner das Verabreichen eines zusätzlichen therapeutischen Mittels an das Individuum umfasst.

7. T-Typ-Calciumkanal-Antagonist zur Verwendung nach Anspruch 6, wobei das zusätzliche therapeutische Mittel ein weiterer T-Typ-Calciumkanal-Inhibitor ist.

8. T-Typ-Calciumkanal-Antagonist zur Verwendung nach Anspruch 7, wobei das zusätzliche therapeutische Mittel ein Antikonvulsivum ist.

9. T-Typ-Calciumkanal-Antagonist zur Verwendung nach Anspruch 8, wobei das Antikonvulsivum aus Acetazolamid, Clobazam, Clonazepam, Eslicarbazepinacetat, Ethosuximid, Lacosamid, Levetiracetam, Nitrazepam, Oxcarbazepin, Perampanel, Piracetam, Phenobarbital, Pregabalin, Primidon, Retigabin, Rufinamid, Valproat, Stiripentol, Tiagabin, Topiramat, Vigabatrin und Zonisamid ausgewählt ist.

10. T-Typ-Calciumkanal-Antagonist zur Verwendung nach einem der Ansprüche 1 bis 9, das ferner das Verabreichen einer zusätzlichen Therapie umfasst, die aus der aus einer ketogenen Diät, Physiotherapie, Ergotherapie, Kommunikationstherapie und Verhaltenstherapie bestehenden Gruppe ausgewählt ist.

## Revendications

1. Antagoniste des canaux calcium de type T pour une utilisation dans un procédé de traitement du syndrome de Dravet chez un sujet, le procédé comprenant une administration au sujet qui en a besoin d'une quantité thérapeutiquement efficace de l'antagoniste des canaux calcium de type T, l'antagoniste des canaux calcium de type T étant le MK-8998 : 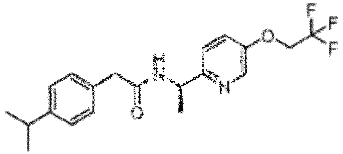 ou un sel pharmaceutiquement acceptable correspondant.

2. Antagoniste des canaux calcium de type T pour une utilisation selon la revendication 1, le traitement comprenant la réduction ou l'amélioration d'au moins un symptôme neurologique chez le sujet.

3. Antagoniste des canaux calcium de type T pour une utilisation selon la revendication 2, le symptôme neurologique comprenant l'un ou plusieurs parmi une crise, l'hyperactivité, l'impulsivité, un comportement autistique, la somnolence, l'insomnie, un retard psychomoteur, une défaillance cognitive liée à l'ataxie, le développement neurologique, un retard développemental et un trouble du comportement.

4. Antagoniste des canaux calcium de type T pour une utilisation selon la revendication 3, le symptôme neurologique comprenant une crise et la crise étant une crise fébrile.

5. Antagoniste des canaux calcium de type T pour une utilisation selon l'une quelconque des revendications 1 à 4, le traitement comprenant l'amélioration de la mémoire à court terme ou de la mémoire de travail du sujet.

6. Antagoniste des canaux calcium de type T pour une utilisation selon l'une quelconque des revendications 1 à 5, comprenant en outre une administration au sujet d'un agent thérapeutique supplémentaire.

7. Antagoniste des canaux calcium de type T pour une utilisation selon la revendication 6, l'agent thérapeutique supplémentaire étant un inhibiteur des canaux calcium de type T supplémentaire.

8. Antagoniste des canaux calcium de type T pour une utilisation selon la revendication 7, l'agent thérapeutique supplémentaire étant un agent anticonvulsif.

9. Antagoniste des canaux calcium de type T pour une utilisation selon la revendication 8, l'agent anticonvulsif étant choisi parmi acétazolamide, clobazam, clonazépam, acétate d'eslicarbazépine, éthosuximide, lacosamide, lévétiracétam, nitrazépam, oxcarbazépine, pérampanel, piracétam, phénobarbital, prégabaline, primidone, rétigabine, rufinamide, valproate, stiripentol, tiagabine, topiramate, vigabatrine et zonisamide.

10. Antagoniste des canaux calcium de type T pour une utilisation selon l'une quelconque des revendications 1 à 9, comprenant en outre une administration d'une thérapie supplémentaire choisie dans le groupe constitué par un régime cétogène, une thérapie physique, une thérapie occupationnelle, une thérapie de communication et une thérapie comportementale.
